# EUROPÄISCHE PATENTSCHRIFT

(11) **EP 0 629 406 B1**
(45) Veröffentlichungstag und Bekanntmachung des Hinweises auf die Patenterteilung: **27.02.2002**
(21) Anmeldenummer: 94107265.4
(22) Anmeldetag: 10.05.1994
(51) Int. Cl.: A61K 38/00, A61K 45/06, A61K 38/58

(54) **Arzneimittel zur Behandlung der Sepsis und des septischen Schocks**
Medicament for the treatment of sepsis or septic shock
Medicament pour le traitement de la septicemie et des chocs septiques

(30) Priorität: 25.05.1993 DE 4317282
(43) Veröffentlichungstag der Anmeldung: 21.12.1994
(73) Patentinhaber: Aventis Behring Gesellschaft mit beschränkter Haftung, 35002 Marburg (DE)
(72) Erfinder: Dickneite, Gerhard, Dr., D-35043 Marburg (DE); Bosslet, Klaus, Dr., D-35037 Marburg (DE)

(56) Entgegenhaltungen:
- DE-A- 4 115 453
- US-A- 4 994 367
- SURG. RES. COMM. Bd. 7 , 1990 Seiten 311 - 18 NAESS ET AL 'Multitherapy. A Treatment Principle to Modify Hemodynamic and Proteolytic Effects of Endotoxin'
- J LAB CLIN MED Bd. 113 , 1989 Seiten 753 - 8 BODE ET AL 'The Use of Thrombin Inhibitors and Aprotinin in the Preservation of Platelets Stored for Transfusion'
- BEHRINGH INST. MITT. Bd. 93 , Dezember 1993 Seiten 299 - 305 DICKNEITE G. 'Influence of C1-Inhibitor on Inflammation, Edema and Shock'
- ARCH INTERN MED Bd. 152 , 1992 Seiten 1381 - 89 BONE C. ROGER 'Modulator of Coagulation. A Critical Appraisal of their Role in Sepsis'
- ACTA ANAESTHOLOGIA SCAND. SUPPL. Bd. 35 , 1991 Seiten 169 - 71 AASEN ET AL 'New Aspects in the Treatment of Septicemia'
- ZENTRALBL CHIR Bd. 118 , 1993 Seiten 482 - 91 BÖHRER ET AL 'Intensivmedizinische Aspekte der Sepsis und des Septischen Multiorganversagens'
- ARCHIVES OF DISEASE IN CHILDHOOD Bd. 68 , Mai 1993 Seiten 621 - 3 HEYDERMAN S. R. 'Sepsis and Intravascular Thrombosis'
- INTENSIVE CARE MED. Bd. 19 , Juli 1993 Seiten S1 - S2 HACK C. E. 'Inhibitor Substitution in Sepsis'
- INTENSIVE CARE MED. Bd. 19 , Juli 1993 MAMMEN E. F. 'Perspectives for the Future'
- THE J. OF INFECT. DISEASES Bd. 151, Nr. 6 , 1985 Seiten 1019 - 27 KALTER ET AL 'Activation and Inhibition of Hageman Factor-Dependent Pathways and the Complement System in Uncomplicated Bacteremia or Bacterial Shock'

## Beschreibung

Die Erfindung betrifft ein Zweikomponenten-System für die Behandlung und Prophylaxe der Sepsis und des septischen Schocks, wobei die Komponenten bestimmt sind, in Kombination miteinander zu wirken, ein Arzneimittel sowie eine Verpakkungseinheit enthaltend beide Komponenten sowie ein Verfahren zu ihrer Herstellung.

Trotz des Fortschrittes in der Antibiotika-Therapie ist die bakterielle Sepsis ein zentrales Problem in der Intensivmedizin, und die Mortalität im septischen Schock ist mit 50 - 70 % nach wie vor unakzeptabel hoch.

Speziell gramnegative Bakterien sind im septischen Krankengut nachgewiesen worden; als Infektionsquelle kommt der Magen-Darm-Trakt, die ableitenden Harnwege, der Respirationstrakt, sowie infizierte Wunden und Verbrennungen in Frage.

Nach der Freisetzung von Endotoxinen (Lipopolysaccharid, LPS) aus der Bakterienzellwand gramnegativer Bakterien kommt es zu einer Bindung von LPS an das "Lipopolysaccharide Binding Protein (LPB), der LPS/LPB Komplex bindet über den CD14-Rezeptor an Makrophagen. Die so stimulierten Makrophagen sezernieren Zytokine wie u. a. TNFα, Interleukin-1 und Interleukin-6. Durch diese Mediatoren werden Granulozyten aktiviert und das Endothel von einem antikoagulatorischen Zustand in einen prokoagulatorischen Zustand überführt. Durch die Expression von Thromboplastin wird der extrinsische Weg der Gerinnung über die Bildung eines Faktor VII/Thromboplastin Komplexes aktiviert. Es resultiert eine Aktivierung des Prothrombinase-Komplexes und damit eine Umwandlung von inaktivem Prothrombin in enzymatisch aktives Thrombin (Faktor IIa). Durch die Spaltung von Fibrinogen in Fibrin resultiert die Bildung von Mikrothromben (disseminierte intravasale Coagulopathie, DIC) im Endstrombett. Die verminderte Durchblutung führt zu einer Sauerstoffunterversorgung und damit zu Organdysfunktionen (multiples Organversagen). Auf der anderen Seite kann LPS direkt den Faktor XII (Hagemann Faktor) durch die Kontaktaktivierung des intrinsischen Gerinnungssystems und somit auch das Kallikrein/Kinin System sowie das Komplementsystem aktivieren. Die Bildung von Bradykinin bewirkt einen Abfall des Blutdruckes und trägt somit ebenfalls zur Ausbildung des septischen Schocks bei.

Es ist bekannt, zur Therapie der Sepsis Antikörper gegen LPS sowie Antikörper, Antagonisten oder lösliche Rezeptoren gegen TNF oder Interleukin-1 einzusetzen.

Thrombin ist die zentrale Protease des Gerinnungssystems, durch ihre Inaktivierung kann die Bildung von Mikrothromben unterdrückt werden.

Antithrombin III ist der physiologische Inhibitor des Thrombins, das Protein mit einem Molekulargewicht von 58 kD kann aus menschlichem Plasma isoliert werden. Antithrombin III reagiert in einem stöchiometrischen Verhältnis mit Thrombin, die Geschwindigkeit der Reaktion wird durch sulfatierte Polysaccharide, speziell Heparin, stark erhöht.

In der Sepsis und im septischen Schock wird ein starker Abfall der Plasmaspiegel von aktivem Antithrombin III beobachtet, was einerseits auf einen erhöhten Verbrauch (Bildung eines Thrombin-Antithrombin Komplexes), andererseits auf eine proteolytische Degradation durch Serin-Proteasen, speziell durch Elastase, die aus polymorphkernigen Granulozyten sezerniert wird, schließen läßt.

Es ist bekannt, daß die Substitution von Antithrombin III bei der Sepsis eingesetzt werden kann (B. Blauhut et al., Thromb. Res. 39, 81-89, 1985).

Ein weiterer, hochspezifischer Inhibitor des Thrombins stellt Hirudin dar. Für Hirudin wurde ebenfalls Wirksamkeit in der experimentellen Sepsis gezeigt (H. Hoffmann et al., Am. Rev. Respir. Dis. 142, 782-788, 1990).

Es war gefunden worden, daß beide Verbindungen die Sepsis-bedingte Mortalitätsrate verringerten und die Überlebenszeit verlängerten.

F. Naess et al. (Surg. Res. Comm., 1990, Vol. 7, pp.311-318) schlägt zur Therapie von Sepsis eine Multitherapie aus einer Kombination 7 verschiedener Wirkstoffe vor, darunter C1 Esterase-Hemmer, AT III und Aprotinin. Ebenso findet sich in R. C. Bone (Arch. Intern. Med., Vol. 152, Juli 1992, pp. 13811389) eine große Liste von etwa 20 Wirkstoffen. Auch R. S. Heyderman (Archives of Disease in Childhood, 1993, Vol. 68, pp. 621-625) enthält lediglich eine Aufzählung verschiedenster Behandlungsvorschläge aus einem Repertoire bzw. einer Basis dreier prinzipieller Vorgehensweisen. In H. Böhrer et al. (Zentralblatt für Chirurgie, 1993, Vol. 118, pp. 482-491) sind schließlich als Therapieansätze nebeneinander die Gabe von C1 Esteraseinhibitor, die pharmakologische Hemmung von Stickoxyd (NO), die Plasmapherese, die Applikation von nicht-steroidalen enzündungshemmenden Substanzen und hochdosiertem Naloxon sowie die Manipulation auf Zytokinebene beschrieben.

Es wurde nun überraschenderweise gefunden, daß sich die antithrombotische Therapie der Sepsis durch die Kombination mit Substanzen, welche nicht auf das Gerinnungssystem wirken, verbessern läßt, und daß diese Kombination zu einer weiteren Reduktion der Mortalität in der Sepsis und im septischen Schock führt.

Als Thrombininhibitoren sind beispielsweise geeignet aus Humanplasma oder gentechnisch hergestelltes Antithrombin III (AT III) sowie Mutanten mit thrombininhibierender Aktivität, natürliches oder gentechnisch hergestelltes Hirudin sowie Mutanten des Hirudin mit thrombin-inhibierender Aktivität oder synthetische Thrombininhibitoren, das heißt, chemisch hergestellte Substanzen, die durch ihre thrombin-inhibierende Wirkungen gekennzeichnet sind.

Als Kombination mit einem antithrombotischen Prinzip sind Substanzen geeignet, welche die Bildung, die Freisetzung, die Plasma- und Gewebsspiegel sowie die Rezeptorbindung von Zytokinen beeinflussen sowie Inhibitoren des Komplements and des Kallikrein/Kinin-Systems.

Geeignete Substanzen sind Inhibitoren, Antagonisten oder lösliche Rezeptoren von Zytokinen oder Antagonisten von Zytokin-Rezeptoren, vorzugsweise der Zytokine TNF (Tumor Necrosis Factor) und IL-1, oder die Fusionsproteine dieser Substanzen mit einem Fc-Teil eines Antikörpers.

Als Zytokin-Inhibitoren und Zytokin-Rezeptoren oder -Antagonisten sind beispielsweise geeignet Substanzen, die die biologische Wirksamkeit von Interleukinl unterdrücken, beispielsweise gentechnisch hergestellter löslicher IL-1-Rezeptor, gentechnisch hergestellter IL-1-Rezeptor oder ein Fc-Fusionsprotein, das IL-1-Rezeptor enthält, Antagonisten des IL-1, d. h. IL-1-ähnliche Polypeptide, die an den Rezeptor binden, aber kein Signal auslösen, Substanzen, die die biologische Wirksamkeit vom Tumor-Nekrose-Faktor (TNF) unterdrükken, beispielsweise gentechnisch hergestellter löslicher TNF-Rezeptor, gentechnisch hergestelltes TNF-Rezeptor oder ein Fc-Fusionsprotein, das TNF-Rezeptor enthält, Antagonisten des TNF, d. h. TNF-ähnliche Polypeptide, die an den Rezeptor binden, aber kein Signal auslösen, oder gentechnisch hergestelltes Interleukin 10 oder seine Mutanten, die an den IL-10 Rezeptor binden und dort ein Signal auslösen. Als Komplement-Inhibitoren oder Kallikrein-Inhibitoren sind beispielsweise geeignet aus Humanplasma gereinigter oder gentechnisch hergestellter Cl-Esterase-Inhibitor (C1INH) sowie seine Mutanten mit Enzym-inhibierender Aktivität, synthetische Komplement-Inhibitoren, das heißt chemisch hergestellte Substanzen, die durch ihre komplement-inhibierende Wirkung charakterisiert sind, natürliches oder gentechnisch hergestelltes Aprotinin oder seine kallikrein-inhibierenden. Mutanten oder synthetische Kallikrein-Inhibitoren, das heißt chemisch hergestellte Substanzen, die durch ihre kallikreininhibierende Wirkung charakterisiert sind.

Besonders bevorzugt sind Kombinationen von Antithrombin III (beispielsweise Kybernin^{R}, Behringwerke AG) oder rec. Hirudin (Behringwerke AG) mit C1 Esterase Inhibitor (beispielsweise Berinert^{R}, Behringwerke AG).
- Dosierungen:: AT III 5 - 1000 U/kg
rec. Hirudin 0.1 - 20 mg/kg
C1INH 5 - 1000 U/kg

Die folgenden Beispiele erläutern die Erfindung näher:

### Beispiel 1

Weibliche CD-Ratten wurden mit einer lethalen Dosis Endotoxin (50 mg/kg, i. v.) behandelt. Es wurden drei Gruppen gebildet, die für 5 Stunden, beginnend bei 15 Minuten vor der Gabe von LPS, intravenös infundiert wurden (1 ml/h). Gruppe 1 erhielt physiologische Kochsalzlösung, Gruppe 2 erhielt 0.17 mg/kg x h rekombinantes Hirudin, Gruppe 3 erhielt die Kombinationstherapie von 0.17 mg/kg x h rekombinantes Hirudin und 100 Units/kg x h C1 Esterase Inhibitor. Tabelle 1 zeigt, daß rec. Hirudin eine deutliche Verlängerung der Überlebensrate im Vergleich zur Kontrolle bewirkt, die Kombinationstherapie mit C1-Esterase-Inhibitor war überraschenderweise der Therapie mit dem Antithrombotikum alleine deutlich überlegen.

**Tabelle 1**

| | Mortalität (% Tote) | |
|---|---|---|
| | 6 h | 10 h |
| 1. Kontrolle (n = 8) | 50 | 88 |
| 2. rec. Hirudin, | 0 | 66 |
| 0.17 mg/kg x h (n = 9) | | |
| 3. rec. Hirudin, | 0 | 20 |
| 0.17 mg/kg x h | | |
| + Cl Inhibitor, | | |
| 100 U/kg x h (n = 10) | | |
| Signifikanzen: 1 -> 2 p < 0.01 1 -> 3 p < 0.01 | | |
| 1 -> 3 p < 0.01 2 -> 3 p < 0.05 | | |

### Beispiel 2

Im gleichen Tiermodell wie in Beispiel 1 wurden zwei Gruppen gebildet: die erste Gruppe erhielt eine Infusion von 37:5 U/Kg x h des aus humanem Plasma gewonnenen Thrombininhibitors Antithrombin III, die zweite Gruppe erhielt zusätzlich noch 125 U/kg x h C1 Esterase Inhibitor. Tabelle 2 zeigt, daß die Kombinationstherapie mit Antithrombin III und C1INH der Monotherapie mit dem Antithrombotikum alleine überlegen war.

**Tabelle 2**

| | | Mortalität nach 8 h (Tote/Gesamt) |
|---|---|---|
| 1. | AT III | 19/30 |
| | 37.5 U/kg x h | |
| 2. | AT III, 37.5 U/kg x h | 11/30 |
| | + C1INH, 125 U/kg x h | |
| Signifikanz: 1 -> 2 p < 0.05 | | |

Es läßt sich mithin zeigen, daß die antithrombotische Therapie mit Antithrombin III und Hirudin sich mit anderen Prinzipien zur Prophylaxe und Therapie der Sepsis und des septischen Schocks kombinieren läßt.

## Patentansprüche

1. Ein gewerblich hergestelltes Zweikomponenten-System für die Behandlung und Prophylaxe der Sepsis und des septischen Schocks, wobei die Komponenten bestimmt sind, in Kombination miteinander zu wirken, und wobei eine Komponente ein Thrombininhibitor und die zweite eine Substanz, welche die Bildung, die Freisetzung, die Plasma- und Gewebsspiegel sowie die Rezeptorbindung von Zytokinen beeinflußt oder ein Inhibitor des Komplements oder des Kallikrein/Kinin-Systems ist.

2. Zweikomponenten-System nach Anspruch 1, **dadurch gekennzeichnet, daß** die Komponenten in einem Arzneimittel enthalten sind.

3. Zweikomponenten-System nach Anspruch 1, **dadurch gekennzeichnet, daß** die Komponenten in einer Verpackungseinheit enthalten sind.

4. Zweikomponenten-System nach Anspruch 1, wobei der Thrombininhibitor ein aus Humanplasma gewonnenes oder gentechnisch hergestelltes Antithrombin III ist.

5. Zweikomponenten-System nach Anspruch 1, wobei der Thrombininhibitor das natürliche aus Blutegeln isolierte oder ein gentechnisch hergestelltes Hirudin oder eine Mutante des Hirudins oder eine synthetische thrombin-inhibierende Substanz ist.

6. Zweikomponenten-System nach Anspruch 1, wobei die zweite Komponente ein gentechnisch hergestellter löslicher Interleukin-1 Rezeptor oder Rezeptor-Antagonist oder sein Fc-Fusionsprotein, ein gentechnisch hergestellter löslicher TNF-Rezeptor oder Rezeptor-Antagonist oder sein Fc-Fusionsprotein oder das gentechnisch hergestellte Interleukin 10, ein Inhibitor des Komplements oder Cl-Esterase-Inhibitor ist.

7. Zweikomponenten-System nach Anspruch 1, wobei die zweite Komponente ein Inhibitor des Kallikrein/Kinin-Systems ist.

8. Zweikomponenten-System nach Anspruch 5, wobei die zweite Komponente natürliches, aus Säugetiergewebe isoliertes oder gentechnisch hergestelltes Antagosan ist.

9. Verfahren zur Herstellung eines Zweikomponenten-Systems für die Behandlung und Prophylaxe der Sepsis und des septischen Schocks, wobei die Komponenten bestimmt sind, in Kombination miteinander zu wirken, und wobei die eine Komponente ein Thrombininhibitor und die zweite ein Zytokin-Antagonisten, Bradykinin- oder Komplement-Inhibitor ist, **dadurch gekennzeichnet, daß** die Komponenten in eine für die Anwendung am Menschen geeignete Darreichungsform gebracht werden.

10. Verwendung eines Thrombininhibitors und eines Zytokin-Antagonisten oder Bradykinin- oder Komplement- Inhibitors in einem Verfahren zur Herstellung eines Arzneimittels zur Behandlung und Prophylaxe der Sepsis und des septischen Schocks.

## Claims

1. A commercially prepared two-component system for the treatment and prophylaxis of sepsis and of septic shock, where the components are intended to act in combination with each other, and where one component is a thrombin inhibitor and the second a substance which influences the formation, the liberation, the plasma and tissue levels, and the receptor binding of cytokines, or is an inhibitor of complement or of the kallikrein/kinin system.

2. The two-component system as claimed in claim 1, wherein the components are contained in a pharmaceutical.

3. The two-component system as claimed in claim 1, wherein the components are contained in a packaging unit.

4. The two-component system as claimed in claim 1, wherein the thrombin inhibitor is an antithrombin III obtained from human plasma or prepared recombinantly.

5. The two-component system as claimed in claim 1, wherein the thrombin inhibitor is the natural hirudin isolated from leaches or is a recombinantly prepared hirudin, or a mutant of hirudin, or a synthetic thrombin-inhibiting substance.

6. The two-component system as claimed in claim 1, wherein the second component is a recombinantly prepared, soluble, interleukin I receptor or receptor antagonist, or its Fc-fusion protein, a recombinantly prepared, soluble, TNF receptor or receptor antagonist, or its Fc-fusion protein, or recombinantly prepared interleukin 10, an inhibitor of complement, or a C1 esterase inhibitor.

7. The two-component system as claimed in claim 1, wherein the second component is an inhibitor of the kallikrein/kinin system.

8. The two-component system as claimed in claim 5, wherein the second component is natural antagosan isolated from mammalian tissue, or is recombinantly prepared antagosan.

9. A process for preparing a two-component system for the treatment and prophylaxis of sepsis and septic shock, where the components are intended to act in combination with each other, and where the one component is a thrombin inhibitor and the second a cytokine antagonist, or an inhibitor of bradykinin or complement, wherein the components are brought into a suitable administration form for use in humans.

10. The use of a thrombin inhibitor and of a cytokine antagonist, or inhibitor of bradykinin or complement, in a process for preparing a pharmaceutical for the treatment and prophylaxis of sepsis and of septic shock.

## Revendications

1. Système à deux composants, fabriqué industriellement, pour le traitement et la prophylaxie de la septicémie et du choc septique, les composants étant destinés à agir en association l'un avec l'autre, et un composant étant un inhibiteur de la thrombine et le second étant une substance influençant la formation, la libération, le taux plasmatique et tissulaire ainsi que la liaison aux récepteurs de cytokines ou un inhibiteur du complément ou du système kinine/kallicréine.

2. Système à deux composants selon la revendication 1, **caractérisé en ce que** les composants sont contenus dans un médicament.

3. Système à deux composants selon la revendication 1, **caractérisé en ce que** les composants sont contenus dans une unité de conditionnement.

4. Système à deux composants selon la revendication 1, dans lequel l'inhibiteur de thrombine est une antithrombine III obtenue à partir de plasma humain ou produite par génie génétique.

5. Système à deux composants selon la revendication 1, dans lequel l'inhibiteur de thrombine est l'hirudine naturelle isolée à partir de sangsues ou une hirudine produite par génie génétique ou un mutant de l'hirudine ou une substance synthétique inhibant la thrombine.

6. Système à deux composants selon la revendication 1, dans lequel le second composant est un récepteur soluble ou antagoniste soluble de récepteur d'interleukine-1, produit par génie génétique, ou sa protéine de fusion Fc, un récepteur soluble ou antagoniste soluble de récepteur de TNF, produit par génie génétique, ou sa protéine de fusion Fc, ou l'interleukine 10 produite par génie génétique, un inhibiteur du complément ou un inhibiteur d'estérase C1.

7. Système à deux composants selon la revendication 1, dans lequel le second composant est un inhibiteur du système kinine/kallicréine.

8. Système à deux composants selon la revendication 1, dans lequel le second composant est l'aprotinine (Antagosan) naturelle, isolée à partir de tissu mammalien ou produite par génie génétique.

9. Procédé pour la préparation d'un système à deux composants pour le traitement et la prophylaxie de la septicémie et du choc septique, les composants étant destinés à agir en association l'un avec l'autre, et un composant étant un inhibiteur de la thrombine et le second étant un antagoniste de cytokine, un inhibiteur de bradykinine ou du complément, **caractérisé en ce qu'**on met les composants sous une forme galénique appropriée à l'utilisation chez l'homme.

10. Utilisation d'un ibiteur de thrombine et d'un antagoniste de cytokine ou inhibiteur de bradykinine ou du complément, dans un procédé pour la fabrication d'un médicament destiné au traitement et à la prophylaxie de la sepfcémie et du choc septique.
